# EUROPÄISCHE PATENTANMELDUNG

(11) **EP 3 888 605 A1**
(43) Veröffentlichungstag der Anmeldung: **06.10.2021**
(21) Anmeldenummer: 21173670.7
(22) Anmeldetag: 15.07.2016
(51) Int. Cl.: A61F 13/00, A61F 15/00

(54) **VERPACKUNG FÜR EIN MEDIZINISCHES PRODUKT**

(30) Priorität: 16.07.2015 DE 102015111582
(62) Teilanmeldung aus: 16742217.9
(71) Anmelder: BSN medical GmbH, 22761 Hamburg (DE)
(72) Erfinder: Wogram, Marco Peter, 22113 Oststeinbek (DE); Casu, Sascha, 21147 Hamburg (DE)
(74) Vertreter: Uexküll & Stolberg

(57) **Zusammenfassung**

Die vorliegende Erfindung betrifft eine Spule für ein Rollenpflaster mit einer oder zwei Seitenscheiben und/oder einen Deckel/Schutzring für ein Rollenpflaster, die/der gegebenenfalls eine Abreißkante aufweist, sowie deren Herstellung. Die Spule bzw. der Deckel/Schutzring umfasst eine antimikrobiell wirksame Substanz, wobei an der äußeren Oberfläche der Spule bzw. des Deckels/Schutzrings Kontakt zwischen der antimikrobiell wirksamen Substanz und der Umgebung vorliegt.

## Beschreibung

Die vorliegende Erfindung betrifft eine Verpackung für ein medizinisches Produkt, deren Herstellung und Verwendung.

### HINTERGRUND DER ERFINDUNG

Die durch Infektionen mit pathogenen Keimen verursachte Sterberate in Deutschland wird im Jahr 2013 auf mehr als 15.000 Personen geschätzt. Eine Vielzahl dieser Infektionen ist auf eine Kontakt-Kontamination mit den Keimen in Arztpraxen und in Krankenhäusern zurückzuführen. Da diese Keime häufig antibiotikaresistent sind, sind Infektionen mit diesen Keimen besonders problematisch, vor allem für bereits geschwächte Patienten. Um eine Infektionsgefahr durch Kontakt-Kontamination zu verringern, werden medizinische Produkte häufig an ihrer Oberfläche mit antimikrobiell wirksamen Substanzen beschichtet. Üblicherweise wird dabei Silber verwendet. Silber weist ein sehr breites Wirkspektrum auch gegen multiresistente Keime auf, wobei bereits geringe Dosen ausreichen. Darüber hinaus werden oftmals auch antimikrobiell wirksame organische Moleküle, wie zum Beispiel Polyhexanid oder Triclosan, verwendet. Ferner sind antimikrobiell wirksame Substanzen auf Kupfer-, Zinn-, Zink- oder Titanbasis bekannt. Weitere vielversprechende Materialien sind Molybdän und/oder Wolfram enthaltende Substanzen, die in Gegenwart von Wasser ein saures Milieu bilden. Derartige Stoffe sind zum Beispiel aus der EP 2 428 118 A2 bekannt.

Wie bereits ausgeführt, sind die medizinischen Produkte selbst häufig mit antimikrobiellen Substanzen ausgerüstet, die Verpackungen dieser Produkte sind hingegen in der Regel nicht antimikrobiell wirksam. Dies führt dazu, dass sich bei häufigem Kontakt mit der Verpackung eine hohe Keimkonzentration auf der Außenseite der Verpackung in unmittelbarer Nähe zum medizinischen Produkt bilden kann. Dies ist insbesondere bei einem Rollenpflaster der Fall, das üblicherweise auf einer Spule vorliegt und mit einem Deckel/Schutzring vor Kontakt mit der Umgebung geschützt wird. Im praktischen Einsatz wird das Rollenpflaster häufig in der Kitteltasche transportiert, wo es in häufigem Kontakt mit der Umgebung, insbesondere den Händen des Behandlungspersonals steht. Ein und dasselbe Rollenpflaster wird üblicherweise für eine Vielzahl von Patienten verwendet. Die somit auf die Spule oder den Deckel/Schutzring übertragenen Keime können dann leicht durch eventuelle Unachtsamkeiten des Behandlungspersonals oder des Patienten auf den Patienten übertragen werden. Dies kann zu schwerwiegenden Infektionen führen. Die Gefahr einer solchen Kontakt-Kontamination könnte durch eine antimikrobielle Ausrüstung der Spule oder des Deckels/Schutzrings erheblich gesenkt, wenn nicht verhindert werden.

Aus der EP 2 113 466 A1 ist eine mit Silber ausgestattete Tube bekannt, die unter anderem zum Verpacken von pharmazeutischen Produkten wie zum Beispiel Heilsalben verwendet werden kann.

Ferner sind aus der EP 1 555 944 B1 verpackte Medizinprodukte bekannt, die eine Verpackung aufweisen, deren Innenseite antimikrobiell beschichtet ist.

### BESCHREIBUNG DER ERFINDUNG

Der Erfindung liegt die Aufgabe zugrunde, eine Verpackung für ein medizinisches Produkt in Form einer Spule für ein Rollenpflaster mit einer oder zwei Seitenscheiben (im Folgenden "Spule" genannt) und/oder eines Deckels/Schutzrings eines Rollenpflasters (im Folgenden "Deckel/Schutzring" genannt), die/der gegebenenfalls eine Abreißkante aufweist, zur Verfügung zu stellen, die/der bei üblichem Gebrauch an ihrer/- seiner äußeren Oberfläche frei von Keimen ist oder zumindest eine reduzierte Keimanzahl im Vergleich zu üblichen, nicht antimikrobiell ausgerüsteten Spulen bzw. Deckeln/Schutzringen eines Rollenpflasters aufweist und diesen keimfreien bzw. keimreduzierten Zustand über einen längeren Zeitraum beibehält. Die Spule und/oder der Deckel/Schutzring, die/der gegebenenfalls eine Abreißkante aufweist, soll dazu an der Außenseite mit einer antimikrobiell wirksamen Substanz ausgerüstet sein, die in Kontakt mit der Umgebung steht/gelangt. Darüber hinaus soll die Spule bzw. der Deckel/Schutzring, die/der gegebenenfalls eine Abreißkante aufweist, möglichst kostengünstig und einfach hergestellt werden können.

Diese Aufgaben werden durch eine Verpackung für ein medizinisches Produkt in Form einer Spule und/oder eines Deckels/- Schutzrings, die/der gegebenenfalls eine Abreißkante aufweist, gelöst, die/der eine antimibikrobiell wirksame Substanz umfasst, wobei an der äußeren Oberfläche der Spule und/oder des Deckels/Schutzrings Kontakt zwischen der antimikrobiell wirksamen Substanz und der Umgebung vorliegt. Ferner werden diese Aufgaben durch ein Verfahren zur Herstellung der erfindungsgemäßen Spule und/oder des erfindungsgemäßen Deckels/Schutzrings, die/der gegebenenfalls eine Abreißkante aufweist, gelöst, bei dem eine antimikrobiell wirksame Substanz mindestens an der äußeren Oberfläche der Spule und/oder des Deckels/- Schutzrings angeordnet wird.

Im Folgenden wird die vorliegende Erfindung mit Bezug auf die Figuren im Detail beschrieben. Die Figuren zeigen dabei lediglich bevorzugte Ausführungsformen und beschränken die Erfindung in keiner Weise.
Fig. 1 zeigt eine Spule für ein Rollenpflaster mit zwei Seitenscheiben und einen Deckel/Schutzring für ein Rollenpflaster im voneinander getrennten Zustand sowie im kombinierten Zustand.
Fig. 2 zeigt eine Spule mit zwei lösbar mit dem Kern verbundenen Seitenscheiben.

### Verpackung

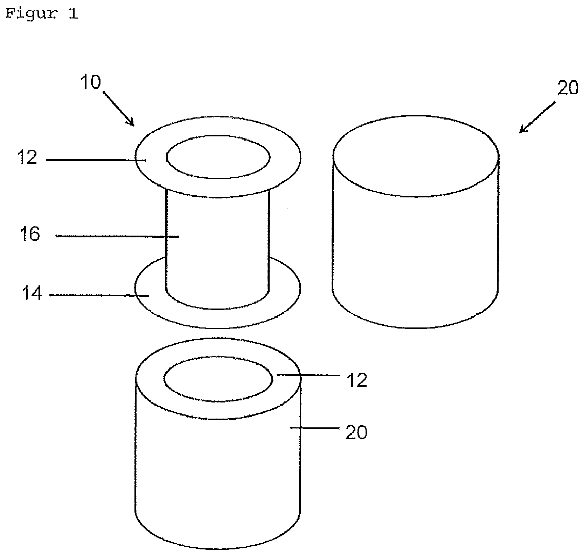

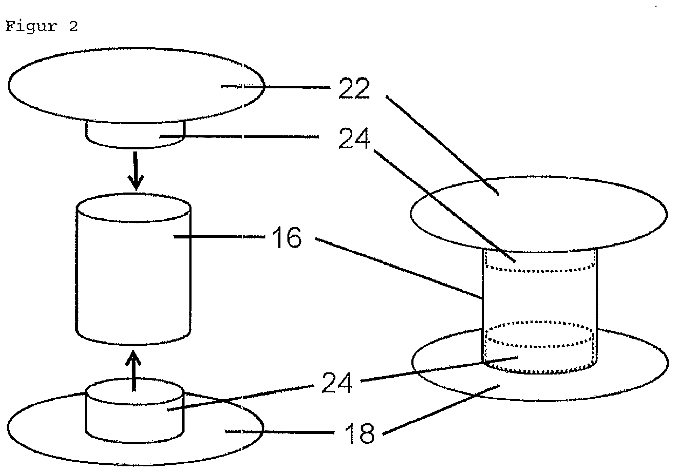

Die Spule 10 und/oder der Deckel/Schutzring 20, die/der gegebenenfalls eine Abreißkante aufweist, umfasst erfindungsgemäß eine antimikrobiell wirksame Substanz, wobei an der äußeren Oberfläche der Spule 10 bzw. des Deckels/Schutzrings 20 Kontakt zwischen der antimikrobiell wirksamen Substanz und der Umgebung vorliegt. Eine bevorzugte Ausführungsform der erfindungsgemäßen Spule weist zwei an einem Kern 16 angebrachte Seitenscheiben 12, 14 auf, wobei die Spule besonders bevorzugt aus einem Stück ("aus einem Guss") gefertigt ist (Fig. 1). Gemäß einer anderen Ausführungsform umfasst die Spule einen Kern 16 und zwei lösbar mit dem Kern verbundene Seitenscheiben 18, 22, die mittels einer Befestigungsvorrichtung 24 auf beide Seiten des Kerns 16 aufgesteckt sind (Fig. 2). Dabei sind die Seitenscheiben und gegebenenfalls auch der Kern antimikrobiell ausgerüstet. Alternativ können die Befestigungsvorrichtung 24 und die Innenseite des Kerns 16 als ineinandergreifende Gewinde ausgestaltet sein, mittels derer die Seitenscheiben 18, 22 an dem Kern 16 befestigt sind. Eine erfindungsgemäße Spule kann auch nur eine an einem Kern angebrachte Seitenscheibe umfassen. In diesem Fall kann die (Schutz) Funktion der anderen (fehlenden) Seitenscheibe von dem Deckel 20 übernommen werden.

Die antimikrobiell wirksame Substanz ist nicht auf bestimmte Substanzen beschränkt. Geeignet sind beispielsweise Metalle, Metallverbindungen, wie zum Beispiel Salze oder Oxide, photokatalytisch aktive Substanzen, wie zum Beispiel Titandioxid oder Siliciumdioxid (Kieselgel), oder organische Verbindungen, wie zum Beispiel Triclosan oder Polyhexanid (PHMB). Weitere geeignete organische Verbindungen umfassen Verbindungen ausgewählt aus der Gruppe der Antistatika, z.B. anionische Antistatika, Hydrophilierungsmittel, oberflächenaktive Stoffe wie Sulfonate und insbesondere sekundäre Alkansulfonate, z.B. C₁₃-C₁₇-Alkansulfonat-Natriumsalze, insbesondere C₁₄-C₁₇-Alkansulfonat-Natriumsalze, sowie Adsorbentien. Die antimikrobiell wirksamen organischen Verbindungen können auch zusammen mit einer oder mehreren anderen antimikrobiell wirksamen Substanzen verwendet werden.

Vorzugsweise umfasst die antimikrobiell wirksame Substanz ein Mitglied der Gruppe bestehend aus Silber, Kupfer, Zinn, Zink, Titan, Molybdän, Wolfram, Verbindungen derselben, einem antimikrobiell wirksamen organischen Molekül, oder einer beliebigen Kombination davon. Bevorzugt umfasst die antimikrobiell wirksame Substanz eine oder mehrere Wolfram- und/oder Molybdänverbindungen.

Die antimikrobielle Wirkung einer Molybdän und/oder Wolfram enthaltenden Substanz liegt in der Bildung von Wasserstoff-Kationen, genauer H₃O⁺, bei Kontakt mit wässrigem Medium begründet. So reagiert zum Beispiel Molybdänoxid mit Wasser zu Molybdänsäure (H₂MoO₄), die wiederum mit Wasser zu H₃O⁺ und MoO₄⁻ oder MoO₄²⁻ reagiert. Die Wasserlöslichkeit der Molybdän und/oder Wolframenthaltenden Substanzen ist dabei so gering, dass die Substanzen quasi nicht verbraucht werden. Somit ist der antimikrobielle Effekt nahezu dauerhaft vorhanden, zumindest aber während des gesamten Benutzungszeitraums der Spule bzw. des Deckels/Schutzrings. Zur Bildung der Wasserstoff-Kationen sind bereits sehr geringe Wassermengen wie z.B. ein nur einige Nanometer dicker Film ausreichend. Das zur Bildung der Wasserstoff-Kationen benötigte Wasser kann beispielsweise aus an Händen befindlichem Schweiß oder der Luftfeuchtigkeit stammen.

Eine Silber enthaltende, antimikrobiell wirksame Substanz umfasst beispielsweise metallisches Silber, wie zum Beispiel kolloidales Silber, ein Silbersalz, wie zum Beispiel Silberchlorid oder Silbernitrat, oder Silberoxid. Eine Titan enthaltende, antimikrobiell wirksame Substanz enthält bevorzugt Titandioxid. Geeignet ist beispielsweise auch eine Silicium enthaltende Verbindung wie Kieselgel. Eine Molybdän enthaltende, antimikrobielle Substanz umfasst bevorzugt eine Verbindung ausgewählt aus der Gruppe bestehend aus MoO₃, MoO₂, ZnMoO₄, Molybdännitrid, Molybdänkarbid, Molybdänsilizid, Molybdänsulfid, oder eine beliebige Kombination davon. Eine Wolfram enthaltende, antimikrobiell wirksame Substanz umfasst bevorzugt eine Verbindung ausgewählt aus der Gruppe bestehend aus Wolframkarbid, Wolframnitrid, Wolframsilizid, WO₃ oder eine beliebige Kombination davon. Ein geeignetes organisches Molekül ist beispielsweise ausgewählt aus der Gruppe bestehend aus Triclosan, Polyhexanid oder einer Mischung davon. Geeignet sind ferner organische Verbindungen ausgewählt aus der Gruppe der Antistatika, z.B. anionische Antistatika, Hydrophilierungsmittel, oberflächenaktive Stoffe wie Sulfonate und insbesondere primäre oder sekundäre, bevorzugt sekundäre Alkansulfonate, z.B. C₁₃-C₁₇-Alkansulfonat-Natriumsalze, insbesondere C₁₄-C₁₇-Alkansulfonat-Natriumsalze, sowie Adsorbentien. Die antimikrobiell wirksamen organischen Verbindungen können auch zusammen mit einer oder mehreren anderen antimikrobiell wirksamen Substanzen verwendet werden. Geeignet ist auch eine Kombination der obengenannte antimikrobiellen Mittel wie beispielsweise eine Kombination von Mo und/oder W mit Zn in Form einer Verbindung derselben oder einer Mischung von Verbindungen derselben, z.B. ZnMoO₄, das in wässriger Umgebung ebenfalls das bereits erwähnte MoO₄²⁻ freisetzt, wahlweise auch in Kombination mit beispielsweise einer der oben genannten organischen Verbindungen, insbesondere einem Antistatikum, beispielsweise einem Sulfonat wie einem primären oder sekundären C₁₃-C₁₇-Alkansulfonat-Natriumsalz.

Die antimikrobiell wirksame Substanz liegt beispielsweise in Form von Partikeln vor. Die Partikelgröße hängt dabei insbesondere von der antimikrobiell wirksamen Substanz ab. Der Fachmann wird die Partikelgröße anhand seines Fachwissens auswählen. Enthält die antimikrobiell wirksame Substanz beispielsweise kolloidales Silber, so liegt dieses bevorzugt in Form von Partikeln mit einer durchschnittlichen Größe (Durchmesser) von weniger als 100 *µ*m, vorzugsweise weniger als 10 *µ*m, bevorzugter weniger als 1 *µ*m, noch bevorzugter 1 bis 500 nm, insbesondere 1 bis 100 nm, vor. Enthält die antimikrobiell wirksame Substanz beispielsweise MoO₂, so liegt dieses bevorzugt in Form von Partikeln mit einer durchschnittlichen Größe von 0,5 bis 10 *µ*m, bevorzugt 2 bis 8 *µ*m, bevorzugter 3 bis 5, am bevorzugtesten etwa 3,6 *µ*m, vor. Enthält die antimikrobiell wirksame Substanz beispielsweise ZnMoO₄, so liegt dieses bevorzugt in Form von Partikeln mit einer durchschnittlichen Größe von 0,5 bis 10 *µ*m, bevorzugt 1 bis 8 *µ*m, bevorzugter 1 bis 5, noch bevorzugter 1 bis 2 *µ*m, am bevorzugtesten etwa 1,5 *µ*m vor. Enthält die antimikrobiell wirksame Substanz beispielsweise MoO₃ und/oder WO₃, so liegt dieses bevorzugt in Form von Partikeln mit einer durchschnittlichen Größe von 5 bis 30 *µ*m, bevorzugt 10 bis 25 *µ*m, bevorzugter 13 bis 20 *µ*m, am bevorzugtesten etwa 16 *µ*m, vor. Die Partikelgröße wird erfindungsgemäß mittels Elektronenmikroskopie (REM oder TEM) bestimmt. Der Fachmann wird dabei die jeweilige Messmethode anhand seines Fachwissens und der Partikelgröße auswählen. Im Grundsatz gilt jedoch, dass TEM eher für kleine Partikel im Nanometerbereich und REM eher für größere Partikel im Mikrometerbereich verwendet wird.

Zur Herstellung der erfindungsgemäßen Spule 10 und/oder des erfindungsgemäßen Deckels/Schutzrings 20, die/der gegebenenfalls eine Abreißkante aufweist, wird als Verpackungsgrundmaterial Papier, Pappe, ein oder mehrere Thermoplaste, ein oder mehrere Duroplaste, ein oder mehrere Elastomere, Metall oder eine Mischung davon verwendet, bevorzugt ein oder mehrere Thermoplaste. Bevorzugt ist ein solcher Thermoplast ausgewählt aus der Gruppe bestehend aus Acrylnitril-Butadien-Styrol, Polyamid, Polylactat, Polymethylmethacrylat, Polycarbonat, Polyethylenterephthalat, Polyethylen, Polypropylen, Polystyrol, Polyetheretherketon, Polyurethan und irgendeiner Kombination davon. Besonders bevorzugt ist der Thermoplast Polystyrol.

Die äußere Oberfläche der Spule 10 bzw. des Deckels/Schutzringes 20 kann vollständig oder teilweise mit der antimikrobiell wirksamen Substanz bedeckt sein.

Die Spule 10 bzw. der Deckel/Schutzring 20 kann dadurch mit der antimikrobiell wirksamen Substanz ausgestattet sein, dass die antimikrobiell wirksame Substanz vor Formgebung der Spule 10 bzw. des Deckels/Schutzringes 20 mit dem Verpackungsgrundmaterial gemischt wurde und die Spule 10 bzw. der Deckel/- Schutzring 20 anschließend aus diesem Gemisch geformt wird. Bevorzugt ist die Spule 10 bzw. der Deckel/Schutzring 20 mittels eines Spritzgussverfahrens geformt. Auf diese Weise liegt antimikrobiell wirksame Substanz an der Oberfläche frei.

In diesem Fall ist die antimikrobiell wirksame Substanz in der Regel in einer Menge von 0,1 bis 10 Gew.-%, bevorzugt 0,1 bis 5 Gew.-%, bevorzugter 0,1 bis 3 Gew.-%, noch bevorzugter 1 bis 2,5 Gew.-%, am bevorzugtesten 2 Gew.-% oder 1,5 Gew.-%, bezogen auf das Gesamtgewicht der Spule 10 bzw. des Deckels/Schutzringes 20 vorhanden. Falls eine antimikrobielle organische Substanz, gegebenenfalls in Kombination mit beispielsweise einer Molybdänverbindung wie ZnMoO₄, verwendet wird, wie beispielsweise ein Antistatikum, liegt die Menge im Bereich von 0,1 bis 10 Gew.-%, bevorzugt 1,0 bis 5 Gew.-% und vorzugsweise 2,5 bis 4,0 Gew.-%, insbesondere bei 3,5 Gew.-%.

Alternativ ist die Spule 10 bzw. der Deckel/Schutzring 20 dadurch mit der antimikrobiell wirksamen Substanz ausgestattet, dass vor der Formgebung der Spule 10 bzw. des Deckels/- Schutzrings 20 keine antimikrobiell wirksame Substanz im Verpackungsgrundmaterial vorhanden ist und nach Formgebung mindestens die äußere Oberfläche der Spule 10 bzw. des Deckels/Schutzrings 20 ganz oder teilweise mit der antimikrobiell wirksamen Substanz bedeckt wird. Dies erfolgt bevorzugt mittels Lackieren, Bedampfen, eines Sprühverfahrens, Sol-Gel-Technik, Bedrucken und/oder eines Tauchverfahrens. Auf diese Weise ist mindestens die äußere Oberfläche ganz oder teilweise mit der antimikrobiell wirksamen Substanz bedeckt.

Es ist auch möglich, beide Ausrüstungsvarianten zu kombinieren.

Auch der Zusatz von färbenden Materialien ist möglich, um Farbgebung zu bewirken. Geeignet sind hier beispielsweise Farbmasterbatche wie die kommerziell von der Fa. Firma Karl Finke GmbH erhältlichen Farbmasterbatche, z.B. Farbmasterbatch (Art. Nr. 626404). Die Mengenbereiche für solche farbgebenden Materialien betragen 0,1 bis 10 Gew.-%, vorzugsweise 1 bis 5 Gew.-%, z.B. 1,8 Gew.-%, 2 Gew.-%, 2,2 Gew.-% und 3,8 Gew.-%.

### Verfahren

Die vorliegende Erfindung betrifft ferner ein Verfahren zur Herstellung der erfindungsgemäßen Spule 10 und/oder des Deckels/Schutzrings 20, die/der gegebenenfalls eine Abreißkante aufweist, bei dem eine antimikrobiell wirksame Substanz mindestens an der äußeren Oberfläche der Spule 10 bzw. des Deckels/Schutzrings 20 angeordnet wird.

Gemäß einer Ausführungsform ist das Verfahren dadurch gekennzeichnet, dass Verpackungsgrundmaterial zur Verfügung gestellt wird und die antimikrobiell wirksame Substanz mit dem Verpackungsgrundmaterial gemischt wird, um ein Masterbatch zu bilden. Anschließend wird das Gemisch aus Verpackungsgrundmaterial und antimikrobiell wirksamer Substanz (Masterbatch) zu einer Spule 10 bzw. einem Deckel/Schutzring 20 geformt, sodass die antimikrobiell wirksame Substanz gleichmäßig verteilt in der Spule 10 bzw. dem Deckel/Schutzring 20 sowie an deren/dessen Oberfläche vorliegt. Dabei besteht mindestens an der äußeren Oberfläche Kontakt zwischen der antimikrobiell wirksamen Substanz und der Umgebung. Erfindungsgemäß werden als Verpackungsgrundmaterial Papier, Pappe, ein oder mehrere Thermoplaste, ein oder mehrere Duroplaste, ein oder mehrere Elastomere, Metall oder irgendeine Mischung davon verwendet. Bevorzugt ist das Verpackungsgrundmaterial aus der Gruppe bestehend aus einem oder mehreren Thermoplasten ausgewählt. Besonders bevorzugt ist das Verpackungsgrundmaterial ausgewählt aus der Gruppe bestehend aus Acrylnitril-Butadien-Styrol, Polyamid, Polylactat, Polymethylmethacrylat, Polycarbonat, Polyethylenterephthalat, Polyethylen, Polypropylen, Polystyrol, Polyetheretherketon, Polyurethan und irgendeiner Kombination davon. Insbesondere ist das Verpackungsgrundmaterial Polystyrol.

Das Masterbatch kann direkt zu einer Spule 10 bzw. einem Deckel/Schutzring 20, die/der gegebenenfalls eine Abreißkante aufweist, geformt werden. Dabei wird die antimikrobiell wirksame Substanz beispielsweise in einer Menge von 0,1 bis 10 Gew.-%, bevorzugt 0,1 bis 5 Gew.-%, bevorzugter 0,1 bis 3 Gew.-%, noch bevorzugter 1 bis 2,5 Gew. am bevorzugtesten 2 Gew.-% oder 1,5 Gew.-%, bezogen auf das Gesamtgewicht des Masterbatches, mit dem Verpackungsgrundmaterial gemischt bzw. bereits bei der Herstellung des Verpackungsgrundmaterials mit einbezogen.

Das Masterbatch kann auch vor der Formgebung mit weiterem Verpackungsgrundmaterial oder weiterer antimikrobiell wirksamer Substanz gemischt werden. Dabei werden die GewichtsVerhältnisse aus antimikrobiell wirksamer Substanz und Verpackungsgrundmaterial so eingestellt, dass die antimikrobiell wirksame Substanz in einer Menge von 0,1 bis 10 Gew.-%, bevorzugt 0,1 bis 5 Gew.-%, bevorzugter 0,1 bis 3 Gew.-%, noch bevorzugter 1 bis 2,5 Gew.-%, am bevorzugtesten 2 Gew.-% oder 1,5 Gew.-%, bezogen auf das Gesamtgewicht der nach der Formgebung erhaltenen Spule 10 bzw. Deckels/Schutzringes 20, die/der gegebenenfalls eine Abreißkante aufweist, in der erhaltenen Spule 10 bzw. dem erhaltenen Deckel/Schutzring 20 vorliegt. Ein solches mit weiterem Verpackungsgrundmaterial (und gegebenenfalls weiterer antimikrobiell wirksamer Substanz) zu mischendes Masterbatch ist konzentrierter und enthält zum Beispiel 10 bis 30 Gew.-%, vorzugsweise 20 Gew.-% der antimikrobiell wirksamen Substanz. Beim Mischen mit dem weiteren Verpackungsgrundmaterial wird dann die gewünschte Konzentration im Endprodukt eingestellt.

Bevorzugt wird die Spule 10 bzw. der Deckel/Schutzring 20 mittels eines Spritzgussverfahrens geformt. In diesem Fall umfasst das Verpackungsgrundmaterial bevorzugt einen oder mehrere Thermoplaste, bevorzugt ein Mitglied der Gruppe bestehend aus Acrylnitril-Butadien-Styrol, Polyamid, Polylactat, Polymethylmethacrylat, Polycarbonat, Polyethylenterephthalat, Polyethylen, Polypropylen, Polystyrol, Polyetheretherketon, Polyurethan und irgendeiner Kombination davon, besonders bevorzugt Polystyrol.

Alternativ ist vor der Formgebung der Spule 10 bzw. des Deckels/Schutzrings 20 keine antimikrobiell wirksame Substanz vorhanden, sodass zunächst eine Spule bzw. ein Deckel/Schutzring gebildet wird, die/der frei von antimikrobiell wirksamer Substanz ist. In diesem Fall wird mindestens die äußere Oberfläche der geformten Spule 10 bzw. Deckels/Schutzrings 20 mit der antimikrobiell wirksamen Substanz mittels Lackieren, Bedampfen, eines Sprühverfahrens, Sol-Gel-Technik, Bedrucken und/oder eines Tauchverfahrens bedeckt, sodass mindestens die äußere Oberfläche ganz oder teilweise mit der antimikrobiell wirksamen Substanz bedeckt ist. Dabei liegt die antimikrobiell wirksame Substanz in einer Menge von 0,1 bis 10 Gew.-%, bevorzugt 0,1 bis 5 Gew.-%, bevorzugter 0,1 bis 3 Gew.-%, am bevorzugtesten 2 Gew.-% in dem Auftragsmedium, zum Beispiel Lack, vor. Auch in diesem Fall wird die Spule 10 bzw. der Deckel/Schutzring 20 aus Papier, Pappe, einem oder mehreren Thermoplasten, einem oder mehreren Duroplasten, einem oder mehreren Elastomeren, Metall oder irgendeiner Mischung davon gefertigt, bevorzugt aus einem oder mehreren Thermoplasten, besonders bevorzugt aus Acrylnitril-Butadien-Styrol, Polyamid, Polylactat, Polymethylmethacrylat, Polycarbonat, Polyethylenterephthalat, Polyethylen, Polypropylen, Polystyrol, Polyetheretherketon, Polyurethan und irgendeiner Kombination davon, insbesondere aus Polystyrol.

Beide Ausrüstungsvarianten können auch kombiniert werden.

Bevorzugt wird die antimikrobiell wirksame Substanz in Form von Partikeln eingesetzt. Alternativ kann die Beschichtung auch partikelfrei, zum Beispiel in Form eines Filmes, vorliegen. Partikelfreie Beschichtungen können beispielsweise mittels Lackieren, Bedampfen, Bedrucken oder Sol-Gel-Verfahren gebildet werden.

Die erfindungsgemäße Spule bzw. der erfindungsgemäße Deckel/- Schutzring ist besonders für die Verwendung zur Verringerung der Infektionsgefahr durch Kontakt-Kontamination, insbesondere in Arztpraxen oder Krankenhäusern, geeignet.

### Ausführungsbeispiele

### Beispiel 1

200 g Polystyrol (High Impact 6540 der Firma Total Petrochemicals) werden mit 800 g MoO₂ (Partikelgröße 3,6 *µ*m) gemischt. Aus dieser Mischung wird mittels Extrusion und anschließender Granulierung ein Masterbatch hergestellt. 25 g des erhaltenen Masterbatches werden mit 975 g Polystyrol gemischt und erneut extrudiert und anschließend granuliert. Das so erhaltene Material wird mittels Spritzgussverfahren zu einer Spule mit zwei Seitenscheiben 10 und einem Deckel/Schutzring 20 geformt.

### Beispiel 2

200 g Polystyrol (High Impact 6540 der Firma Total Petrochemicals) werden mit 800 g WO₃ (Partikelgröße 16 *µ*m) gemischt. Aus dieser Mischung wird mittels Extrusion und anschließender Granulierung ein Masterbatch hergestellt. 25 g des erhaltenen Masterbatches werden mit 975 g Polystyrol gemischt und erneut extrudiert und anschließend granuliert. Das so erhaltene Material wird mittels Spritzgussverfahren zu einer Spule mit zwei Seitenscheiben 10 und einem Deckel/Schutzring 20 geformt.

### Beispiel 3

200 g Polystyrol (High Impact 6540 der Firma Total Petrochemicals) werden mit 400 g MoO₂ (Partikelgröße 3,6 *µ*m) und 400 g WO₃ (Partikelgröße 16 *µ*m) gemischt. Aus dieser Mischung wird mittels Extrusion und anschließender Granulierung ein Masterbatch hergestellt. 25 g des erhaltenen Masterbatches werden mit 975 g Polystyrol gemischt und erneut extrudiert und anschließend granuliert. Das so erhaltene Material wird mittels Spritzgussverfahren zu einer Spule mit zwei Seitenscheiben 10 und einem Deckel/Schutzring 20 geformt.

### Beispiel 4

1000 g Polystyrol (High Impact 6540 der Firma Total Petrochemicals) wird mit 250 g ZnMoO₄ (Partikelgröße 1-1,5 µm) gemischt. Aus dieser Mischung wird mittels Extrusion und anschließender Granulierung ein Masterbatch hergestellt. 375 g des erhaltenen Masterbatches werden mit 4350 g Polystyrol (High Impact 6540 der Firma Total Petrochemicals), 175 g Antistatikum (Atmer 190 der Firma Croda; CAS 85711-69-9; EINECS 288-330-3) und 60 g Farbmasterbatch (Art.Nr. 626404 der Firma Karl Finke GmbH) gemischt und mittels Spritzgussverfahren zu einer Spule mit zwei Seitenscheiben 10 und einem Deckel/- Schutzring 20 geformt.

### Beispiel 5

1000 g Polystyrol (High Impact 6540 der Firma Total Petrochemicals) wird mit 250 g ZnMoO₄ (Partikelgröße 1-1,5 µm) gemischt. Aus dieser Mischung wird mittels Extrusion und anschließender Granulierung ein Masterbatch hergestellt. 625 g des erhaltenen Masterbatches werden mit 4100 g Polystyrol (High Impact 6540 der Firma Total Petrochemicals), 175 g Antistatikum (Atmer 190 der Firma Croda; CAS 85711-69-9; EINECS 288-330-3) und 60 g Farbmasterbatch (Art.Nr. 626404 der Firma Karl Finke GmbH) gemischt und mittels Spritzgussverfahren zu einer Spule mit zwei Seitenscheiben 10 und einem Deckel/- Schutzring 20 geformt.

### Beispiel 6

1000 g Polystyrol (High Impact 6540 der Firma Total Petrochemicals) wird mit 250 g ZnMoO₄ (Partikelgröße 1-1,5 µm) gemischt. Aus dieser Mischung wird mittels Extrusion und anschließender Granulierung ein Masterbatch hergestellt. 625 g des erhaltenen Masterbatches werden mit 4160 g Polystyrol (High Impact 6540 der Firma Total Petrochemicals) und 175 g Antistatikum (Atmer 190 der Firma Croda; CAS 85711-69-9; EINECS 288-330-3) gemischt und mittels Spritzgussverfahren zu einer Spule mit zwei Seitenscheiben 10 und einem Deckel/- Schutzring 20 geformt.

### Beispiel 7

1000 g Polystyrol (High Impact 6540 der Firma Total Petrochemicals) wird mit 250 g ZnMoO₄ (Partikelgröße 1-1,5 µm) gemischt. Aus dieser Mischung wird mittels Extrusion und anschließender Granulierung ein Masterbatch hergestellt. 375 g des erhaltenen Masterbatches werden mit 4350 g Polystyrol (High Impact 6540 der Firma Total Petrochemicals), 175 g Antistatikum (Heco Stat 290 der Firma Hecoplast GmbH; CAS 97489-15-1 ; EINECS 307-055-2) und 60 g Farbmasterbatch (Art.Nr. 626404 der Firma Karl Finke GmbH) gemischt und mittels Spritzgussverfahren zu einer Spule mit zwei Seitenscheiben 10 und einem Deckel/Schutzring 20 geformt.

In allen obigen Beispielen kann alternativ auch das kommerziell erhältliche Polystyrol "High Impact, Empera 524N" der Firma Ineos Nova verwendet werden.

Die antimikrobielle Wirksamkeit der erhaltenen Produkte gegenüber Staphylococcus aureus und Pseudomonas aeruginosa wird wie in "Materials Science and Engineering: C, Volume 32, Issue 1, 1 January 2012, Pages 47-54, Absatz 2.3" beschrieben bestimmt.

Die Wirksamkeit kann auch gemäß ISO 22196 gegen Staphylococcus aureus und E. coli gezeigt werden, wobei folgende Abwandlungen vorgenommen werden:

| ISO 22196 | Deviation |
|---|---|
| Prüfkörper: flach 50 ± 2 mm x 50 ± 2 mm | hergestellte Spule |
| bedeckte Testfläche: 40 ± 2 mm x 40 ± 2 mm | Seitenscheibe der hergestellten Spule |
| Inkubationszeit: 24h | 3h; 6h; 24h |

Die Zytotoxizität wird mittels MTT-Test wie in "Materials Science and Engineering: C, Volume 32, Issue 1, 1 January 2012, Pages 47-54, Absatz 2.4" beschrieben bestimmt.

## Patentansprüche

1. Verpackung für ein medizinisches Produkt in Form einer Spule (10) für ein Rollenpflaster mit einer oder zwei Seitenscheiben (12, 14) und/oder eines Deckels/Schutzrings eines Rollenpflasters (20) ist, die/der gegebenenfalls eine Abreißkante aufweist,
**dadurch gekennzeichnet, dass**
die Verpackung eine antimikrobiell wirksame Substanz umfasst und an der äußeren Oberfläche der Verpackung Kontakt zwischen der antimikrobiell wirksamen Substanz und der Umgebung vorliegt.

2. Verpackung für ein medizinisches Produkt nach Anspruch 1, **dadurch gekennzeichnet, dass** die antimikrobiell wirksame Substanz ein Mitglied der Gruppe bestehend aus Silber, Kupfer, Zinn, Zink, Titan, Molybdän, Wolfram, Silicium, Verbindungen derselben, einem antimikrobiell wirksamen organischen Molekül, oder eine beliebige Kombination davon umfasst.

3. Verpackung für ein medizinisches Produkt nach einem der vorhergehenden Ansprüche, **dadurch gekennzeichnet, dass** die antimikrobiell wirksame Substanz in einer Menge von 0,1 bis 10 Gew.-%, bevorzugt 0,1 bis 5 Gew.-%, bevorzugter 0,1 bis 3 Gew.-%, noch bevorzugter 1 bis 2,5 Gew.-%, am bevorzugtesten 2 oder 1,5 Gew.-%, bezogen auf das Gesamtgewicht der Verpackung, vorhanden ist.

4. Verpackung für ein medizinisches Produkt nach einem der vorhergehenden Ansprüche, **dadurch gekennzeichnet, dass** die antimikrobiell wirksame Substanz eine Zink-, Molybdän- und/oder Wolframverbindung umfasst und gegebenenfalls außerdem eine organische Verbindung, insbesondere ein Antistatikum umfasst, vorzugsweise MoO₂, WO₃ oder ZnMoO₄, bevorzugt ZnMoO₄ zusammen mit einem Antistatikum, insbesondere einem Sulfonat, bevorzugt einem sekundären C₁₃-C₁₇-Alkansulfonat-Natriumsalz.

5. Verpackung für ein medizinisches Produkt nach einem der vorhergehenden Ansprüche, **dadurch gekennzeichnet, dass** die Verpackung Papier, Pappe, einen oder mehrere Thermoplaste, einen oder mehrere Duroplaste, ein oder mehrere Elastomere, Metall, oder eine Mischung daraus umfasst.

6. Verpackung für ein medizinisches Produkt nach einem der vorhergehenden Ansprüche, **dadurch gekennzeichnet, dass** die äußere Oberfläche der Verpackung vollständig oder teilweise mit der antimikrobiell wirksamen Substanz bedeckt ist.

7. Verfahren zur Herstellung der Verpackung gemäß einem der vorhergehenden Ansprüche, bei dem eine antimikrobiell wirksame Substanz mindestens an der äußeren Oberfläche der Verpackung angeordnet wird.

8. Verfahren zur Herstellung der Verpackung nach Anspruch 7, **dadurch gekennzeichnet, dass**
a1) Verpackungsgrundmaterial zur Verfügung gestellt wird;
b1) die antimikrobiell wirksame Substanz mit dem Verpackungsgrundmaterial gemischt wird, sodass ein Masterbatch gebildet wird;
c1) das Masterbatch zu der Verpackung geformt wird, sodass die antimikrobiell wirksame Substanz gleichmäßig verteilt in der gesamten Verpackung vorliegt und mindestens an der äußeren Oberfläche der Verpackung Kontakt zwischen der wirksamen Substanz und der Umgebung vorliegt.

9. Verfahren zur Herstellung der Verpackung nach Anspruch 8, bei dem nach Schritt b1) und vor Schritt c1) das erhaltene Masterbatch mit weiterem Verpackungsgrundmaterial und/oder antimikrobiell wirksamer Substanz gemischt wird.

10. Verfahren zur Herstellung der Verpackung nach Anspruch 7, **dadurch gekennzeichnet, dass**
a2) eine Verpackung für ein medizinisches Produkt zur Verfügung gestellt wird, die frei von antimikrobiell wirksamer Substanz ist, und
b2) mindestens die äußere Oberfläche der Verpackung vollständig oder teilweise mit einer antimikrobiell wirksamen Substanz beschichtet wird,
sodass mindestens an der äußeren Oberfläche der Verpackung Kontakt zwischen der wirksamen Substanz und der Umgebung vorliegt.

11. Verfahren nach Anspruch 10, **dadurch gekennzeichnet, dass** die äußere Oberfläche der Verpackung in Schritt b2) mittels Lackieren, Bedampfen, einem Sprühverfahren, einem Tauchverfahren, Bedrucken oder einem Sol-Gel-Verfahren beschichtet wird.

12. Verfahren zur Herstellung einer Verpackung nach Anspruch 8 oder 9, das nach Schritt c1) ferner einen Schritt d1) umfasst, bei dem mindestens die äußere Oberfläche der erhaltenen Verpackung zusätzlich mit einer antimikrobiell wirksamen Substanz beschichtet wird.

13. Verfahren zur Herstellung einer Verpackung nach Anspruch 12, **dadurch gekennzeichnet, dass** die erhaltene Verpackung in Schritt d1) mittels Lackieren, Bedampfen, eines Sprühverfahrens, eines Tauchverfahrens, Bedrucken oder eines Sol-Gel-Verfahrens beschichtet wird.

14. Verfahren zur Herstellung einer Verpackung nach einem der Ansprüche 7 bis 13 **dadurch gekennzeichnet, dass** die antimikrobiell wirksame Substanz ein Mitglied der Gruppe bestehend aus Silber, Kupfer, Zinn, Zink, Titan, Silicium, Molybdän, Wolfram, Verbindungen derselben, einem antimikrobiell wirksamen organischen Molekül oder einer beliebigen Kombination davon umfasst.

15. Verpackung gemäß einem der Ansprüche 1 bis 6 bzw. hergestellt durch ein Verfahren gemäß einem der Ansprüche 7 bis 14 zur Verwendung in der Verringerung der Infektionsgefahr durch Kontakt-Kontamination.
